# EUROPEAN PATENT APPLICATION

(11) **EP 3 205 300 A1**
(43) Date of publication of application: **16.08.2017**
(21) Application number: 15861024.6
(22) Date of filing: 10.11.2015
(51) Int. Cl.: A61B 18/00

(54) **VIBRATION-GENERATING UNIT, VIBRATING BODY UNIT, AND ULTRASONIC TREATMENT TOOL**

(30) Priority: 18.11.2014 JP 2014233613
(71) Applicant: Olympus Corporation, Hachioji-shi, Tokyo 192-8507 (JP)
(72) Inventor: YOSHIMINE, Hideto, Hachioji-shi, Tokyo 192-8507 (JP)
(74) Representative: Gunzelmann, Rainer
(86) International application number: PCT/JP2015/081594
(87) International publication number: WO 2016/080242

(57) **Abstract**

A front mass of a vibration generating unit included a flange portion supported, and a block portion provided on a proximal side with respect to the flange portion. A distal end of the element unit abuts on a proximal end of the block portion. Ultrasonic vibration generated in a piezoelectric element is transmitted, thereby vibrating the front mass in a predetermined frequency region where a referential vibration anti-node, which is one of vibration anti-nodes located on the proximal side with respect to the flange portion, is located at a boundary position between the block portion and the element unit or in the vicinity of the boundary position.

## Description

### Technical Field

The present invention relates to a vibration generating unit including an elements unit (a piezoelectric element) to which electric power is supplied so as to generate ultrasonic vibration. Furthermore, the present invention relates to a vibrating body unit including the vibration generating unit, and an ultrasonic treatment instrument including the vibrating body unit.

### Background Art

In Patent Literature 1, there is disclosed an ultrasonic treatment instrument to treat a treatment target of a biological tissue or the like by use of ultrasonic vibration. In this ultrasonic treatment instrument, there is provided an elements unit including piezoelectric elements to which electric power is supplied so as to generate the ultrasonic vibration. The generated ultrasonic vibration is transmitted through a vibration transmitting member (a probe) to a treatment section provided in a distal portion of the vibration transmitting member. A distal end of the elements unit including the piezoelectric elements abuts on a proximal end of a front mass (a distal-side fixed portion). In the front mass, there are provided a flange portion supported by a transducer case, and a horn that enlarges an amplitude of the ultrasonic vibration. In the front mass, the distal end of the elements unit abuts on the flange portion, and the horn is continuous with a distal side of the flange portion.

### Citation List

### Patent Literature

Patent Literature 1: Jpn. Patent Publication No. 4918565

### Summary of Invention

### Technical Problem

In such an ultrasonic treatment instrument as in Patent Literature 1 mentioned above, an elements unit (piezoelectric elements) is different in acoustic impedance from a front mass, and at a boundary position between the elements unit and the front mass, the acoustic impedance to ultrasonic vibration transmitted toward a distal side changes. Furthermore, in a state where the elements unit and the front mass are vibrated by the ultrasonic vibration, a vibration node of the ultrasonic vibration is located in a flange portion supported by a transducer case or in the vicinity of the flange portion. Consequently, in a constitution where a distal end of the elements unit abuts on the flange portion, the boundary position between the elements unit and the front mass at which the acoustic impedance changes is located at the vibration node of the ultrasonic vibration or in the vicinity of the vibration node, and a distance from a vibration anti-node of the ultrasonic vibration to the boundary position is large. The boundary position between the elements unit and the front mass is located at the vibration node, at which stress due to the ultrasonic vibration is large, or in the vicinity of the vibration node, whereby an amplitude of the ultrasonic vibration changes due to the change of the acoustic impedance at the boundary position (an amplitude in the front mass is larger than an amplitude in the elements unit).

Here, characteristics of the piezoelectric elements of the elements unit change due to deterioration with time, a heat sterilization and the like. When the characteristics of the piezoelectric element change, the acoustic impedance of the elements unit also changes from a manufacturing time, and there changes an enlargement ratio (a transformation ratio) of the amplitude of the ultrasonic vibration at the boundary position between the elements unit and the front mass. Due to the change of the enlargement ratio of the amplitude at the boundary position, a vibration velocity (the amplitude) in a treatment section of a vibration transmitting member changes. Due to the change of the vibration velocity in the treatment section, a treatment performance in the treatment changes.

The present invention has been developed in view of the above problems, and an object thereof is to provide a vibration generating unit in which an influence of change of characteristics of piezoelectric elements onto a vibration velocity in a vibration transmitting member (a treatment section) is decreased. Another object of the present invention is to provide a vibrating body unit and an ultrasonic treatment instrument each including the vibration generating unit.

### Solution to Problem

To solve above mentioned problems, according to one aspect of the invention, a vibration generating unit including: a front mass which includes a flange portion supported by a housing case, and a block portion provided on a proximal side with respect to the flange portion, the front mass being capable of transmitting ultrasonic vibration; and an element unit which includes a piezoelectric element to which electric power is supplied so as to generate the ultrasonic vibration, and whose distal end abuts on a proximal end of the block portion, the element unit being configured to transmit the ultrasonic vibration generated in the piezoelectric element from the proximal side to a distal side through the front mass, thereby vibrating the front mass in a predetermined frequency region where a referential vibration anti-node, which is one of vibration anti-nodes located on the proximal side with respect to the flange portion, is located at a boundary position between the block portion and the element unit or in the vicinity of the boundary position.

### Advantageous Effects of Invention

According to the present invention, a vibration generating unit, in which an influence of change of characteristics of piezoelectric elements onto a vibration velocity in a vibration transmitting member (a treatment section) is decreased, can be provided. Further, a vibrating body unit and an ultrasonic treatment instrument, each including the vibration generating unit, can be provided.

### Brief Description of Drawings

FIG. 1 is a schematic view showing an ultrasonic treatment system according to a first embodiment;
FIG. 2 is a cross-sectional view schematically showing a constitution of a transducer unit according to the first embodiment;
FIG. 3 is a schematic view showing a constitution of a vibration generating unit according to the first embodiment, and explaining a state where a vibrating body unit longitudinally vibrates in a predetermined frequency range;
FIG. 4 is a schematic view showing a constitution of a vibration generating unit according to a first modification, and explaining a state where a vibrating body unit longitudinally vibrates in a predetermined frequency range;
FIG. 5 is a schematic view showing a constitution of a vibration generating unit according to a second modification, and explaining a state where a vibrating body unit longitudinally vibrates in a predetermined frequency range;
FIG. 6 is a schematic view showing a constitution of a vibration generating unit according to a third modification, and explaining a state where a vibrating body unit longitudinally vibrates in a predetermined frequency range; and
FIG. 7 is a schematic view showing a constitution of a vibration generating unit according to a fourth modification, and explaining a state where a vibrating body unit longitudinally vibrates in a predetermined frequency range.

### Description of Embodiments

### (First Embodiment)

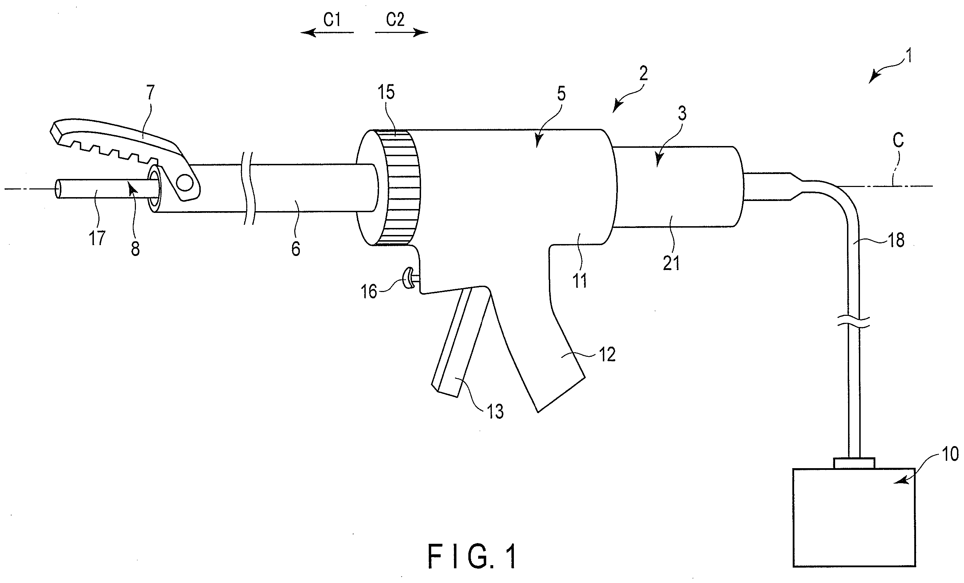

A first embodiment of the present invention will be described with reference to FIG. 1 to FIG. 3.

FIG. 1 is a view showing an ultrasonic treatment system 1 of the present embodiment. As shown in FIG. 1, the ultrasonic treatment system 1 includes an ultrasonic treatment instrument 2. The ultrasonic treatment instrument 2 has a longitudinal axis C. Here, a direction parallel to the longitudinal axis C (a direction along the longitudinal axis C) is defined as a longitudinal axis direction. Furthermore, one side of the longitudinal axis direction is a distal side (an arrow C1 side of FIG. 1) and a side opposite to the distal side is a proximal side (an arrow C2 side of FIG. 1).

The ultrasonic treatment instrument 2 includes a transducer unit 3, a held unit 5 that is holdable by an operator or the like, a sheath 6, a jaw (a gripping member) 7, and a vibration transmitting member (a distal-side vibration transmitting member) 8. The held unit 5 includes a held main body portion 11 extending along the longitudinal axis C, a fixed handle 12 extending from the held main body portion 11 toward a certain direction intersecting the longitudinal axis C, and a movable handle 13 rotatably attached to the held main body portion 11. The movable handle 13 rotates relative to the held main body portion 11, whereby the movable handle 13 opens or closes to the fixed handle 12. The distal side of the held main body portion 11 is coupled with a rotary operation knob 15 that is a rotary operation input section. The rotary operation knob 15 is rotatable around the longitudinal axis C relative to the held main body portion 11. Furthermore, an energy operation button 16 that is an energy operation input section is attached to the held main body portion 11.

The sheath 6 is coupled with the held unit 5 in a state of being inserted into the rotary operation knob 15 and the holding main body portion 11 from the distal side. Furthermore, the jaw 7 is rotatably attached to a distal portion of the sheath 6. The vibration transmitting member 8 extends from the inside of the held main body portion 11 through the inside of the sheath 6 toward the distal side. In the present embodiment, a central axis of the vibration transmitting member 8 coincides with the longitudinal axis C, and the vibration transmitting member 8 extends from its proximal end to its distal end along the longitudinal axis C. A treatment section 17 is provided in a distal portion of the vibration transmitting member 8. The vibration transmitting member 8 is inserted through the sheath 6 in a state where the treatment section 17 projects from a distal end of the sheath 6 toward the distal side. The movable handle 13 that is an opening and closing operation input section performs an opening motion or a closing motion relative to the fixed handle 12, whereby a movable portion (not shown) of the sheath 6 moves along the longitudinal axis C, and the jaw 7 rotates. When the jaw 7 rotates, the jaw 7 performs its opening motion or closing motion relative to the treatment section 17 of the vibration transmitting member 8. Furthermore, the sheath 6, the jaw 7 and the vibration transmitting member 8 are rotatable integrally with the rotary operation knob 15 around the longitudinal axis C relative to the held main body portion 11.

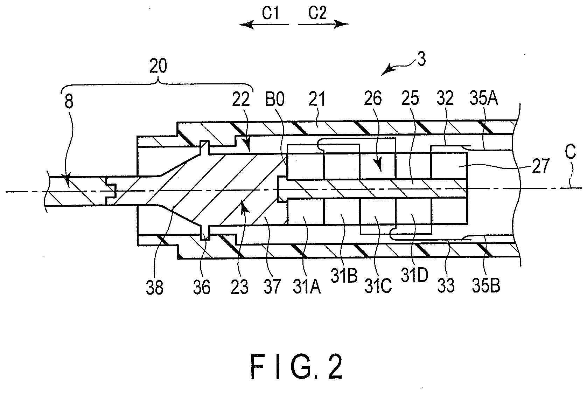

FIG. 2 is a view showing a constitution of the transducer unit 3. As shown in FIG. 1 and FIG. 2, the transducer unit 3 includes a transducer case 21 forming an exterior of the transducer unit 3. The transducer case 21 is coupled with the held unit 5 in a state of being inserted from the proximal side into the held main body portion 11. Further inside the held main body portion 11, the transducer case 21 is separably coupled with the sheath 6. One end of a cable 18 is connected to the transducer case 21. In the ultrasonic treatment system 1, the other end of the cable 18 is separably connected to an energy source unit 10. Here, the energy source unit 10 is, for example, a medical electric power source device (an energy control device), and includes an electric power source, a conversion circuit (which are not shown in the drawing) and others. Further in the energy source unit 10, a controller (not shown) that controls an output of electric power is constituted of a processor including a central processing unit (CPU) or an application specific integrated circuit (ASIC), and a storage (not shown) such as a memory is disposed.

Further in the transducer unit 3, a vibration generating unit 22 is disposed inside the transducer case 21. That is, in the present embodiment, the transducer case 21 is a housing case that houses the vibration generating unit 22 therein. The vibration generating unit 22 is attached to the transducer case 21. The vibration generating unit 22 includes a front mass (a distal-side fixed portion) 23. In the present embodiment, a central axis of the front mass 23 coincides with the longitudinal axis C, and the front mass 23 extends from its proximal end to its distal end along the longitudinal axis C. The distal end of the front mass 23 forms a distal end of the vibration generating unit 22. Inside the held main body portion 11, the distal end of the front mass 23 is separably connected to the proximal end of the vibration transmitting member 8. The front mass 23 is connected to the vibration transmitting member 8, whereby the vibration transmitting member 8 is coupled with the distal side of the vibration generating unit 22. It is to be noted that in a state where the vibration transmitting member 8 is coupled with the vibration generating unit 22, the vibration generating unit 22 is rotatable integrally with the vibration transmitting member 8 around the longitudinal axis C relative to the held main body portion 11.

In the vibration generating unit 22, a pillar-like bolt portion (an elements attaching portion) 25 is connected to a proximal side of the front mass 23. In the present embodiment, a central axis of the bolt portion 25 coincides with the longitudinal axis C, and the bolt portion 25 extends from its proximal end to its distal end along the longitudinal axis C. The proximal end of the bolt portion 25 forms a proximal end of the vibration generating unit 22. The front mass 23 and the bolt portion 25 are made of a material such as titanium (Ti) which is capable of transmitting ultrasonic vibration. It is to be noted that in the present embodiment, the front mass 23 and the bolt portion 25 are separate members, but the front mass 23 and the bolt portion 25 may integrally be formed as one member.

Furthermore, an elements unit 26 and a back mass (a proximal-side fixed portion) 27 are attached to the bolt portion 25. The elements unit 26 and the back mass 27 are formed into a ring shape, and the bolt portion (the elements attaching portion) 25 are inserted into the elements unit 26 and the back mass 27 in this order, whereby the elements unit 26 and the back mass 27 are attached to the bolt portion 25. Therefore, the elements unit 26 and the back mass 27 which are attached to the bolt portion 25 are arranged on an outer peripheral side of the bolt portion 25. The back mass 27 is made of a material such as superduralumin (A2024) which is capable of transmitting the ultrasonic vibration.

The elements unit 26 has a proximal end and a distal end, and extends from the proximal end to the distal end along the longitudinal axis C. A distal end of the back mass 27 abuts on the proximal end of the elements unit 26, and the distal end of the elements unit 26 abuts on the proximal end of the front mass 23. Therefore, the back mass 27 abuts on the elements unit 26 from the proximal side and the front mass 23 abuts on the elements unit 26 from the distal side. Consequently, the elements unit 26 is sandwiched between the back mass (the proximal-side fixed portion) 27 and the front mass (the distal-side fixed portion) 23 in the longitudinal axis direction parallel to the longitudinal axis C (along the longitudinal axis C).

The elements unit 26 includes (four in the present embodiment) piezoelectric elements 31A to 31D, a first electrode member 32 and a second electrode member 33. In the longitudinal axis direction of the vibration generating unit 22, each of the piezoelectric elements 31A to 31D is sandwiched between the first electrode member 32 and the second electrode member 33. One end of an electric wire portion 35A is connected to the first electrode member 32, and one end of an electric wire portion 35B is connected to the second electrode member 33. The electric wire portions 35A and 35B extend through the inside of the cable 18, and the other end of the electric wire portion 35A and the other end of the electric wire portion 35B are electrically connected to the electric power source and the conversion circuit (which are not shown in the drawing) of the energy source unit 10.

Further inside the held unit 5, a switch portion (not shown) is disposed. An opened or closed state of the switch portion switches in response to input of an energy operation with the energy operation button 16. The switch portion is electrically connected to the controller (not shown) of the energy source unit 10 via a signal path portion (not shown) extending through the transducer unit 3 and the inside of the cable 18. The controller detects the opened or closed state of the switch portion, thereby detecting the input of the energy operation with the energy operation button 16. When the input of the energy operation is detected, the electric power is output from the energy source unit 10. The electric power (AC electric power) is output from the energy source unit 10, whereby a voltage is applied between the first electrode member 32 and the second electrode member 33. Due to the voltage applied between the first electrode member 32 and the second electrode member 33, a current (an alternating current) flows through the piezoelectric elements 31A to 31D each sandwiched between the first electrode member 32 and the second electrode member 33, and each of the respective piezoelectric elements 31A to 31D converts the current into the ultrasonic vibration. That is, the electric power is supplied to the piezoelectric elements 31A to 31D, thereby generating the ultrasonic vibration.

An acoustic impedance Z of the piezoelectric elements 31A to 31D is higher than an acoustic impedance Z of the front mass 23 and the back mass 27. Here, the acoustic impedance Z is a product of a cross-sectional area S perpendicular to the longitudinal axis C and a characteristic impedance ζ in a vibrating body unit 20. The characteristic impedance ζ is a physical property determined by a material that forms a component, and the characteristic impedance ζ has a value peculiar to each material (substance). The characteristic impedance ζ is a value determined on the basis of a density ρ of the material and a propagation velocity c of sound in the material (i.e., the density ρ and Young's modulus E of the material). The piezoelectric elements 31A to 31D are made of ceramics such as lead zirconate titanate (PZT) which is a material whose characteristic impedance ζ is higher than that of the front mass 23 and the back mass 27. Consequently, in the piezoelectric elements 31A to 31D, the acoustic impedance Z is higher than that of the front mass 23 and the back mass 27.

The generated ultrasonic vibration is transmitted from the proximal side toward the distal side, and transmitted from the elements unit 26 through the front mass 23. Then, the ultrasonic vibration is transmitted from the front mass 23 to the vibration transmitting member 8, and in the vibration transmitting member 8, the ultrasonic vibration is transmitted toward the treatment section 17. The treatment section 17 treats a treatment target of a biological tissue by use of the transmitted ultrasonic vibration. That is, the vibration generating unit 22 and the vibration transmitting member 8 form the vibrating body unit 20 that vibrates due to the ultrasonic vibration, and the vibrating body unit 20 extends from the proximal end of the vibration generating unit 22 to the distal end of the vibration transmitting member 8. In a state where the ultrasonic vibration is transmitted toward the treatment section 17, the vibrating body unit 20 performs a longitudinal vibration whose vibrating direction is parallel to the longitudinal axis C (the longitudinal axis direction). In the present embodiment, the proximal end of the bolt portion. 25 (a proximal end of the back mass 27) forms a proximal end of the vibrating body unit 20, and the distal end of the vibration transmitting member 8 forms a distal end of the vibrating body unit 20. That is, the ultrasonic vibration generated in the piezoelectric elements 31A to 31D is transmitted through the front mass 23, whereby the vibrating body unit 20 including the front mass 23 and the elements unit 26 vibrates (longitudinally vibrates).

In the front mass 23, a flange portion (a portion to be supported) 36 supported by the transducer case (the housing case) 21 is provided. The vibration generating unit 22 is attached to the transducer case 21 in a state where the flange portion 36 is supported by an inner peripheral portion of the transducer case 21. Furthermore, in the front mass 23, a block portion (a spacer block) 37 is disposed on a proximal side of the flange portion 36. The block portion 37 has a proximal end and a distal end, and extends from the proximal end to the distal end along the longitudinal axis C. The proximal end of the block portion 37 forms the proximal end of the front mass 23. In the present embodiment, the block portion 37 is continuous from a proximal end of the flange portion 36 to the distal end of the elements unit 26 along the longitudinal axis C. Therefore, the distal end of the elements unit 26 abuts on the proximal end of the block portion 37. An abutment portion between the elements unit 26 and the block portion 37 becomes a boundary position B0 between the elements unit 26 and the block portion 37 (the front mass 23).

Furthermore, in the front mass 23, there is formed a tapered horn (a cross-sectional area decreasing portion) 38 in which a cross-sectional area perpendicular to the longitudinal axis C decreases toward the distal side. In the present embodiment, the horn 38 is continuous with a distal side of the flange portion 36. Consequently, in the present embodiment, the flange portion 36 is formed at a proximal end (a horn vibration input end) of the horn 38. Therefore, the horn 38 is located on the distal side with respect to the boundary position B0 between the elements unit 26 and the block portion 37. In a state where the ultrasonic vibration generated in the piezoelectric elements 31A to 31D is transmitted through the front mass 23 toward the distal side, an amplitude of the ultrasonic vibration is enlarged by the horn 38. It is to be noted that in the present embodiment, the distal end of the vibration generating unit 22 is located on the distal side with respect to a distal end (a horn vibration output end) of the horn 38.

Next, there will be described function and effects of the vibration generating unit 22, the vibrating body unit 20 and the ultrasonic treatment instrument 2 of the present embodiment. In a case of performing a treatment by use of the ultrasonic treatment instrument 2, the sheath 6, the jaw 7 and the vibration transmitting member 8 are inserted into a body cavity such as an abdominal cavity in a state where the held unit 5 is held. Then, the treated target of the biological tissue or the like is disposed between the jaw 7 and the treatment section 17 of the vibration transmitting member 8. In this state, the movable handle 13 performs the closing motion relative to the fixed handle 12, and the jaw 7 is closed to the treatment section 17, thereby gripping the treated target between the jaw 7 and the treatment section 17. The energy operation is input with the energy operation button 16 in the state where the treated target is gripped, whereby the electric power is output from the energy source unit 10 and the output electric power is supplied to the piezoelectric elements 31A to 31D of the vibration generating unit 22. Consequently, the ultrasonic vibration is generated in the piezoelectric elements 31A to 31D (the elements unit 26). Then, the generated ultrasonic vibration is transmitted through the front mass 23 to the vibration transmitting member 8, and in the vibration transmitting member 8, the ultrasonic vibration is transmitted toward the treatment section 17. Consequently, the vibrating body unit 20 constituted of the vibration generating unit 22 and the vibration transmitting member 8 performs the longitudinal vibration whose vibrating direction is parallel to the longitudinal axis C. The treatment section 17 longitudinally vibrates in the state where the treated target is gripped between the jaw 7 and the treatment section 17, thereby generating frictional heat between the treatment section 17 and the treated target. By the frictional heat, the treated target is coagulated and simultaneously incised.

In the treatment, the controller of the energy source unit 10 adjusts a frequency of the current of the electric power supplied to the piezoelectric elements 31A to 31D, a current value, a voltage value and the like. Furthermore, the vibrating body unit 20 is designed in a state of transmitting the ultrasonic vibration generated in the piezoelectric elements 31A to 31D through the front mass 23 and the vibration transmitting member 8 to the treatment section 17 to vibrate at a referential resonance frequency Frref (e.g., 47 kHz). In the vibrating body unit 20, the vibration generating unit 22 including the expensive piezoelectric elements 31A to 31D is subjected to a heat sterilization or the like after used, and is reused. On the other hand, the vibration transmitting member 8 is discarded after used.

Here, in the vibration transmitting member 8 and the front mass 23 which are made of titanium, unevenness occurs in physical properties (especially the Young's modulus E) of the material for each component in a manufacturing process. For example, the unevenness occurs in the physical properties of the material of each vibration transmitting member 8, and hence in the vibrating body unit 20, a resonance frequency Fr in a vibrating state changes in accordance with the physical properties of the material of the vibration transmitting member 8 connected to the vibration generating unit 22. Furthermore, in accordance with the physical properties of the material of the front mass 23 for use, the resonance frequency Fr in the vibrating state changes. That is, in the vibrating body unit 20, unevenness occurs in the resonance frequency Fr of the vibration in accordance with the physical properties of the vibration transmitting member 8 and the front mass 23, and the vibrating body unit does not necessarily vibrate at the referential resonance frequency Frref. Therefore, due to the ultrasonic vibration generated in the piezoelectric elements 31A to 31D, the vibrating body unit 20 vibrates (longitudinally vibrates) in a predetermined frequency region Δf that is not less than a minimum resonance frequency Frmin (e.g., 46 kHz) and not more than a maximum resonance frequency Frmax (e.g., 48 kHz). It is to be noted that the referential resonance frequency Frref is included in the predetermined frequency region Δf. As described above, in the vibrating body unit 20 constituted of the vibration generating unit 22 and the vibration transmitting member 8, a dimension and the like are determined so that the unit vibrates in the predetermined frequency range Δf including the referential resonance frequency Frref, and a frequency or the like of the current to be supplied to the piezoelectric elements 31A to 31D is also adjusted so that the vibrating body unit 20 vibrates in the predetermined frequency range Δf including the referential resonance frequency Frref.

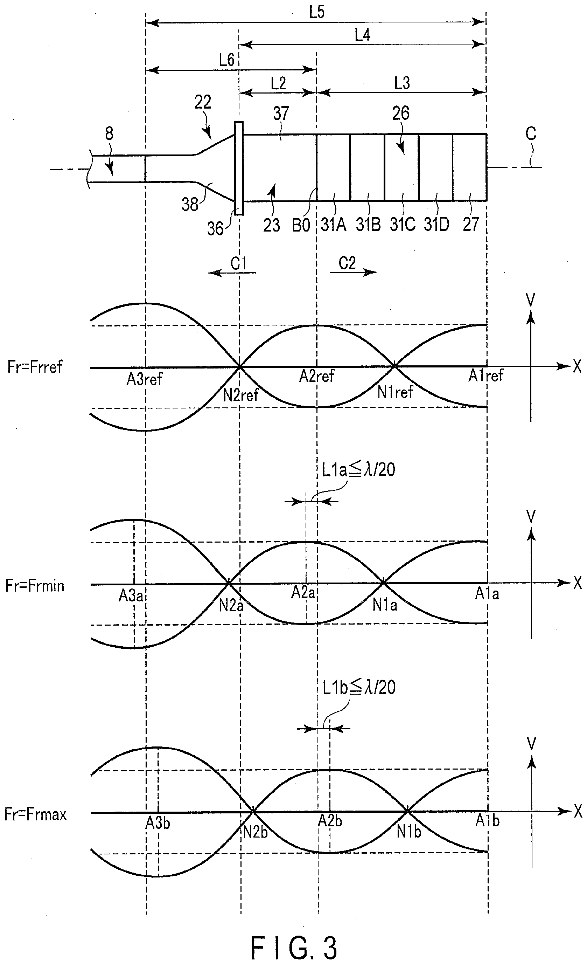

FIG. 3 is a view explaining the longitudinal vibration (the vibration) in the vibration generating unit 22 in a state where the vibrating body unit 20 longitudinally vibrates in the predetermined frequency range Δf. FIG. 3 shows a graph of a state of longitudinally vibrating at the referential resonance frequency Frref, a state of longitudinally vibrating at the minimum resonance frequency Frmin and a state of longitudinally vibrating at the maximum resonance frequency Frmax. In these graphs, the abscissa indicates a position (X) in the longitudinal axis direction and the ordinate indicates an amplitude (V) of the longitudinal vibration. In the state where the vibrating body unit 20 longitudinally vibrates, the distal end and proximal end of the vibrating body unit 20 become free ends. Consequently, one of vibration anti-nodes of the ultrasonic vibration (the longitudinal vibration) is located at the proximal end of the vibrating body unit 20 (the proximal end of the vibration generating unit 22), and one of the vibration anti-nodes of the ultrasonic vibration is located at the distal end of the vibrating body unit 20 (the distal end of the vibration transmitting member 8). As shown in FIG. 3, in the state where the vibrating body unit 20 longitudinally vibrates in the predetermined frequency region Δf, a vibration anti-node A1 (denoted with A1ref, A1a or A1b in FIG. 3) that is one of the vibration anti-nodes of the longitudinal vibration is located at the proximal end of the vibration generating unit 22 (the proximal end of the bolt portion 25). In the present embodiment, the vibration anti-node A1 is the most-proximal vibration anti-node located most proximally among the vibration anti-nodes of the ultrasonic vibration.

Here, the vibration anti-node located distally as much as a half wavelength (λ/2) of the ultrasonic vibration (the longitudinal vibration) from the vibration anti-node A1 is defined as a vibration anti-node A2 (denoted with A2ref, A2a or A2b in FIG. 3), and the vibration anti-node located distally as much as 1 wavelength (λ) of the ultrasonic vibration from the vibration anti-node A1 is defined as a vibration anti-node A3 (denoted with A3ref, A3a or A3b in FIG. 3). The vibration anti-node A2 is located secondly proximally among the vibration anti-nodes of the ultrasonic vibration (the longitudinal vibration), and the vibration anti-node A3 is located thirdly proximally among the vibration anti-nodes of the ultrasonic vibration. Furthermore, a vibration node located distally as much as a 1/4 wavelength (λ/4) of the ultrasonic vibration from the vibration anti-node A1 is defined as a vibration node N1 (denoted with N1ref, N1a or N1b in FIG. 3), and a vibration node located distally as much as a 1/4 wavelength (λ/4) of the ultrasonic vibration from the vibration anti-node A2 is defined as a vibration node N2 (denoted with N2ref, N2a or N2b in FIG. 3). The vibration node N1 is located most proximally among the vibration nodes of the ultrasonic vibration, and the vibration node N2 is located secondly proximally among the vibration nodes of the ultrasonic vibration.

Furthermore, a wavelength λ of the ultrasonic vibration (the longitudinal vibration) in a state where the vibrating body unit 20 vibrates at the referential resonance frequency Frref is defined as a referential wavelength λref. When the resonance frequency Fr decreases from the referential resonance frequency Frref, the wavelength λ of the ultrasonic vibration (the longitudinal vibration) increases from the referential wavelength λref. Therefore, in the vibration in the predetermined frequency range Δf, the wavelength λ becomes a maximum wavelength λmax when the vibrating body unit 20 vibrates at the minimum resonance frequency Frmin. On the other hand, when the resonance frequency Fr increases from the referential resonance frequency Frref, the wavelength λ of the ultrasonic vibration (the longitudinal vibration) decreases from the referential wavelength λref. Therefore, in the vibration in the predetermined frequency range Δf, the wavelength λ becomes a minimum wavelength λmin when the vibrating body unit 20 vibrates at the maximum resonance frequency Frmax.

In a state where the vibrating body unit 20 vibrates at the referential resonance frequency Frref, the vibration node N2ref (N2) is located in the flange portion 36 of the front mass 23 in the longitudinal axis direction. Furthermore, in a state where the vibrating body unit 20 vibrates at the minimum resonance frequency Frmin, the vibration node N2a slightly shifts from the flange portion 36 to the distal side, and in a state where the vibrating body unit 20 vibrates at the maximum resonance frequency Frmax, the vibration node N2b slightly shifts from the flange portion 36 to the proximal side. However, in each of the state where the vibrating body unit 20 vibrates at the minimum resonance frequency Frmin and the state where the vibrating body unit 20 vibrates at the maximum resonance frequency Frmax, the shift of the vibration node N2 (N2a or N2b) from the flange portion 36 is micro. Therefore, in a state where the vibrating body unit 20 vibrates in the predetermined frequency range Δf that is not less than the minimum resonance frequency Frmin and is not more than the maximum resonance frequency Frmax, the vibration node N2 at which the amplitude is zero is located in the flange portion 36 or in the vicinity of the flange portion 36. Consequently, in a state where the vibrating body unit 20 vibrates in the predetermined frequency range Δf including the referential resonance frequency Frref, the amplitude of the ultrasonic vibration in the flange portion 36 is zero, or the flange portion 36 hardly vibrates. Therefore, the vibration generating unit 22 is firmly attached to the transducer case 21 in the flange portion 36, and transmission of the ultrasonic vibration from the vibration generating unit 22 through the flange portion 36 to the transducer case 21 is effectively prevented.

In the state where the vibrating body unit 20 vibrates at the referential resonance frequency Frref, the vibration anti-node A2ref (A2) that is a referential vibration anti-node is located at the boundary position B0 between the elements unit 26 and the front mass 23 (the block portion 37) in the longitudinal axis direction. Furthermore, in the state where the vibrating body unit 20 vibrates at the minimum resonance frequency Frmin, the wavelength λ becomes the maximum wavelength λmax, and hence the vibration anti-node A2a slightly shifts from the boundary position B0 to the distal side, and in the state where the vibrating body unit 20 vibrates at the maximum resonance frequency Frmax, the wavelength λ becomes the minimum wavelength λmin, and hence the vibration anti-node A2b slightly shifts from the flange portion 36 to the proximal side. However, in each of the state where the vibrating body unit 20 vibrates at the minimum resonance frequency Frmin and the state where the vibrating body unit 20 vibrates at the maximum resonance frequency Frmax, the shift of the vibration anti-node A2 (A2a or A2b) from the boundary position B0 is micro. Therefore, in the state where the vibrating body unit 20 vibrates in the predetermined frequency range Δf that is not less than the minimum resonance frequency Frmin and is not more than the maximum resonance frequency Frmax, the vibration anti-node A2 at which stress due to the ultrasonic vibration is zero is located at the boundary position B0 or in the vicinity of the boundary position B0.

Actually, in the state where the vibrating body unit 20 vibrates at the minimum resonance frequency Frmin, a distance L1a from the boundary position B0 between the elements unit 26 and the front mass 23 (the block portion 37) to the vibration anti-node (the referential vibration anti-node) A2a in the distal side is not more than a 1/20 wavelength (λ/20) of the ultrasonic vibration in the predetermined frequency range Δf. Furthermore, in the state where the vibrating body unit 20 vibrates at the maximum resonance frequency Frmax, a distance L1b from the boundary position B0 between the elements unit 26 and the front mass 23 to the vibration anti-node (the referential vibration anti-node) A2b in the proximal side is not more than a 1/20 wavelength (λ/20) of the ultrasonic vibration in the predetermined frequency range Δf. Therefore, in the state where the vibrating body unit 20 including the front mass 23 and the elements unit 26 vibrates in the predetermined frequency range Δf, a distance (L1) from the boundary position B0 to the vibration anti-node A2 in the longitudinal axis direction is zero or is not more than the 1/20 wavelength (λ/20) of the ultrasonic vibration.

In the state where the vibrating body unit 20 vibrates in the predetermined frequency range Δf, the vibration anti-node (the referential vibration anti-node) A2 located at the boundary position B0 between the front mass 23 and the elements unit 26 or in the vicinity of the boundary position B0 is one of the vibration anti-nodes (A1 and A2) positioned on the proximal side with respect to the flange portion 36, and is closest to the flange portion 36 among the vibration anti-nodes (A1 and A2) positioned on the proximal side with respect to the flange portion 36. Furthermore, in the state where the vibrating body unit 20 vibrates in the predetermined frequency range Δf, the vibration node N2 located distally as much as the 1/4 wavelength (λ/4) of the ultrasonic vibration from the vibration anti-node A2 as described above is located in the flange portion 36 or in the vicinity of the flange portion 36. Therefore, in the state where the vibrating body unit 20 vibrates in the predetermined frequency range Δf, a distance L2 between the flange portion 36 and the boundary position B0 in the longitudinal axis direction is the same or about the same as the 1/4 wavelength of the ultrasonic vibration. Furthermore, in the state where the vibrating body unit 20 vibrates in the predetermined frequency range Δf, a distance L3 between the proximal end of the vibration generating unit 22 and the boundary position B0 in the longitudinal axis direction is the same or about the same as the half wavelength of the ultrasonic vibration. Therefore, in the state where the vibrating body unit 20 vibrates in the predetermined frequency range Δf, a distance L4 between the proximal end of the vibration generating unit 22 and the flange portion 36 in the longitudinal axis direction is the same or about the same as a 3/4 wavelength (3λ/4) of the ultrasonic vibration.

Furthermore, according to the present embodiment, in the state where the vibrating body unit 20 vibrates at the referential frequency Frref, the vibration anti-node A3ref (A3) is located at the distal end of the vibration generating unit 22. Furthermore, in the state where the vibrating body unit 20 vibrates at the minimum resonance frequency Frmin, the vibration anti-node A3a slightly shifts from the distal end of the vibration generating unit 22 to the distal side, and in the state where the vibrating body unit 20 vibrates at the maximum resonance frequency Frmax, the vibration anti-node A3b slightly shifts from the distal end of the vibration generating unit 22 to the proximal side. However, in each of the state where the vibrating body unit 20 vibrates at the minimum resonance frequency Frmin and the state where the vibrating body unit 20 vibrates at the maximum resonance frequency Frmax, the shift of the vibration anti-node A3 (A3a or A3b) from the distal end of the vibration generating unit 22 is micro. Therefore, in the state where the vibrating body unit 20 vibrates in the predetermined frequency range Δf that is not less than the minimum resonance frequency Frmin and is not more than maximum resonance frequency Frmax, the vibration anti-node A3 is located at the distal end of the vibration generating unit 22 or in the vicinity of the distal end of the vibration generating unit 22.

The vibration anti-node A3 is located distally as much as the 1 wavelength (λ) of the ultrasonic vibration from the vibration anti-node A1 located at the proximal end of the vibration generating unit 22. Consequently, in the state where the vibrating body unit 20 including the front mass 23 and the elements unit 26 vibrates in the predetermined frequency region Δf, a total length (a distance between the distal end and the proximal end) L5 of the vibration generating unit 22 in the longitudinal axis direction is the same or about the same as 1 wavelength of the ultrasonic vibration, and is larger than the 3/4 wavelength of the ultrasonic vibration. Furthermore, in the state where the vibrating body unit 20 vibrates in the predetermined frequency region Δf, a distance L6 from the boundary position B0 between the front mass 23 and the elements unit 26 to the distal end of the vibration generating unit 22 (the distal end of the front mass 23) in the longitudinal axis direction is the same or about the same as the half wavelength of the ultrasonic vibration. Therefore, in the state where the vibrating body unit 20 vibrates in the predetermined frequency region Δf, a distance from the vibration anti-node (the referential vibration anti-node) A2 located at the boundary position B0 or in the vicinity of the boundary position B0 to the distal end of the front mass 23 in the longitudinal axis direction is the same or about the same as the half wavelength of the ultrasonic vibration, and is larger than the 1/4 wavelength of the ultrasonic vibration.

Furthermore, the horn 38 of the front mass 23 is located between the distal end of the vibration generating unit 22 and the boundary position B0 in the longitudinal axis direction, and hence in the state where the vibrating body unit 20 vibrates in the predetermined frequency region Δf, the horn 38 is located between the vibration anti-node A2 and the vibration anti-node A3. Therefore, in the state where the vibrating body unit 20 vibrates in the predetermined frequency region Δf, the vibration anti-nodes (A1 to A3) in which the stress due to the ultrasonic vibration is zero are not located in the horn 38, and in the horn 38, the stress due to the ultrasonic vibration acts. Consequently, in the horn 38 in which the cross-sectional area perpendicular to the longitudinal axis C decreases toward the distal side (in a transmitting direction of the ultrasonic vibration), the amplitude of the ultrasonic vibration is enlarged. An enlargement ratio (a transformation ratio) ε1 indicating the amplitude at the distal end (the vibration output end) of the horn 38 to the amplitude at the proximal end (the vibration input end) of the horn 38 increases, as the stress of the ultrasonic vibration in a position in which the horn 38 is disposed increases. In the present embodiment, the vibration node N2 at which the stress due to the ultrasonic vibration is locally maximized is located in the vicinity of the proximal end (the flange portion 36) of the horn 38, and hence the enlargement ratio ε1 in the horn 38 increases. It is to be noted that when the vibrating body unit 20 is vibrated at the certain resonance frequency Fr that is not included in the predetermined frequency region Δf, it is possible to locate, in the horn 38, one of the vibration anti-nodes at which the stress is zero. However, in this case, irrespective of a change ratio (a decrease ratio) of a cross-sectional area in the horn 38, the amplitude of the ultrasonic vibration is not enlarged in the horn 38, and the enlargement ratio ε1 becomes a minimum value of 1.

Furthermore, the piezoelectric elements 31A to 31D (the elements unit 26) are made of a material in which the characteristic impedance ζ is high as compared with the front mass 23 (the block portion 37), and the acoustic impedance Z is high as compared with the front mass 23. Consequently, at the boundary position B0 between the elements unit 26 and the block portion 37, the acoustic impedance Z relative to the ultrasonic vibration to be transmitted toward the distal side changes. In a case where the stress due to the ultrasonic vibration acts at a position such as the boundary position B0 at which the acoustic impedance Z changes, the amplitude of the ultrasonic vibration changes at a position at which the acoustic impedance Z changes (a position at which at least one of the physical properties and the cross-sectional area S changes). According to the present embodiment, in the front mass 23 located on the distal side (on the side of the transmitting direction of the ultrasonic vibration) with respect to the boundary position B0, the acoustic impedance Z decreases as compared with the elements unit 26 located on the proximal side with respect to the boundary position B0. Consequently, in the case where the stress due to the ultrasonic vibration acts at the boundary position B0, the amplitude of the ultrasonic vibration is enlarged at the boundary position B0, and the amplitude in the block portion 37 is larger than the amplitude in the elements unit 26. Furthermore, an enlargement ratio (a transformation ratio) ε2 of the amplitude of the ultrasonic vibration at the boundary position B0 (i.e., the ratio of the amplitude of the ultrasonic vibration in the block portion 37 relative to the amplitude of the ultrasonic vibration in the elements unit 26) increases, as the stress due to the ultrasonic vibration which acts at the boundary position B0 increases.

According to the present embodiment, in the state where the vibrating body unit 20 vibrates in the predetermined frequency region Δf, the vibration anti-node (the referential vibration anti-node) A2 at which the stress due to the ultrasonic vibration is zero is located at the boundary position B0 or in the vicinity of the boundary position B0. Consequently, the stress due to the ultrasonic vibration is zero or the stress hardly acts at the boundary position B0 between the elements unit 26 and the block portion 37. In a case where the stress due to the ultrasonic vibration is zero or the stress hardly acts at the boundary position B0, irrespective of a change ratio of the acoustic impedance Z at the boundary position B0, the enlargement ratio ε2 of the amplitude at the boundary position B0 is small value, and is a minimum value of 1 or a value close to 1. Therefore, in the state where the vibrating body unit 20 vibrates in the predetermined frequency region Δf, irrespective of the change ratio of the acoustic impedance Z at the boundary position B0, the amplitude of the ultrasonic vibration hardly changes (is hardly enlarged) at the boundary position B0.

The piezoelectric elements 31A to 31D of the elements unit 26 deteriorate with an elapse of time, and is subjected to a heat sterilization such as autoclave sterilization after the use in the treatment. Consequently, characteristics of the piezoelectric elements 31A to 31D change due to the deterioration with time, the heat sterilization treatment or the like. When the characteristics of the piezoelectric elements 31A to 31D change, the acoustic impedance Z (the acoustic characteristic impedance ζ) also changes from a manufacturing time in the elements unit 26, and the change ratio of the acoustic impedance Z between the elements unit 26 and the block portion 37 (the front mass 23) (i.e., at the boundary position B0) also changes. However, in the present embodiment, irrespective of the change ratio of the acoustic impedance Z at the boundary position B0 as described above, the enlargement ratio ε2 of the amplitude at the boundary position B0 decreases to the minimum value of 1 or to the value close to 1. Consequently, also in a case where the characteristics of the piezoelectric elements 31A to 31D change from the manufacturing time, the enlargement ratio ε2 of the amplitude at the boundary position B0 hardly changes from the manufacturing time in the state where the vibrating body unit 20 vibrates in the predetermined frequency region Δf. The enlargement ratio ε2 of the amplitude at the boundary position B0 does not change from the manufacturing time, and hence in the state where the vibrating body unit 20 vibrates in the predetermined frequency region Δf, the amplitude of the ultrasonic vibration in the front mass 23 (the block portion 37) also hardly changes from the manufacturing time, and a vibration velocity (the amplitude) in the treatment section 17 of the vibration transmitting member 8 also hardly changes from the manufacturing time. Therefore, in the present embodiment, an influence of the change of the characteristics of the piezoelectric elements 31A to 31D onto the vibration velocity in the vibration transmitting member 8 (the treatment section 17) is decreased. Consequently, also in a case where the characteristics of the piezoelectric elements 31A to 31D change due to the deterioration with time, the heat treatment or the like, the vibration velocity in the treatment section 17 does not change from the manufacturing time, and the treatment can be performed with a stable treatment performance.

Furthermore, in the front mass 23, the unevenness occurs in the physical properties (especially the Young's modulus E) of the material for each component in the manufacturing process as described above. Consequently, in the front mass 23 for use in the vibration generating unit 22, the acoustic impedance Z (the acoustic characteristic impedance ζ) changes with each component. Therefore, the change ratio of the acoustic impedance Z at the boundary position B0 between the front mass 23 and the elements unit 26 changes in accordance with the front mass 23 for use in the vibration generating unit 22. That is, in accordance with the physical properties of the front mass 23, the change ratio of the acoustic impedance Z at the boundary position B0 is uneven with each vibration generating unit 22. However, in the present embodiment as described above, irrespective of the change ratio of the acoustic impedance Z at the boundary position B0, the enlargement ratio ε2 of the amplitude at the boundary position B0 decreases to the minimum value of 1 or to the value close to 1. Consequently, also in the case where the change ratio of the acoustic impedance Z at the boundary position B0 is uneven with each product, the unevenness of the enlargement ratio ε2 at the boundary position B0 with each product decreases, and variability of the vibration velocity in the treatment section 17 of each product also decreases. That is, in the present embodiment, the influence of the physical properties of the front mass 23 onto the vibration velocity of the treatment section 17 decreases. In consequence, irrespective of the physical properties (the Young's modulus E, etc.) of the front mass 23, a stabilized treatment performance can be acquired also in the ultrasonic treatment instrument 2 in which any type of vibration generating unit 22 is used.

### (Modifications)

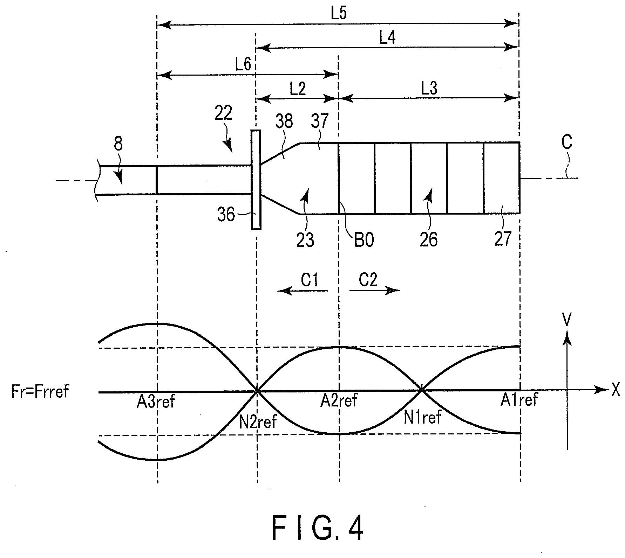

It is to be noted that in the first embodiment, the flange portion 36 is provided at the proximal end (the vibration input end) of the horn 38, but it is not limited to this embodiment. For example, as shown as a first modification in FIG. 4, a flange portion 36 to be supported by a transducer case 21 may be provided at a distal end (a vibration output end) of a horn 38. FIG. 4 shows a graph of a state where a vibrating body unit 20 longitudinally vibrates at a referential resonance frequency Frref in a predetermined frequency range Δf, in addition to a constitution of a vibration generating unit 22. In this graph, the abscissa indicates a position (X) in a longitudinal axis direction and the ordinate shows an amplitude (V) of the longitudinal vibration.

As shown in FIG. 4, also in the present modification, a vibration node N2 of ultrasonic vibration is located in the flange portion 36 in a state where the vibrating body unit 20 vibrates at the referential resonance frequency Frref. Then, in a state where the vibrating body unit 20 vibrates in a predetermined frequency region Δf that is not less than a minimum resonance frequency Frmin and is not more than a maximum resonance frequency Frmax, the vibration node N2 is located in the flange portion 36 or in the vicinity of the flange portion 36. Furthermore, also in the present modification, a vibration anti-node A2 of the ultrasonic vibration is located at a boundary position B0 between a front mass 23 (a block portion 37) and an elements unit 26, in the state where the vibrating body unit 20 vibrates at the referential resonance frequency Frref. Then, in the state where the vibrating body unit 20 vibrates in the predetermined frequency region Δf, the vibration anti-node A2 is located at the boundary position B0 or in the vicinity of the boundary position B0. That is, in the state where the vibrating body unit 20 vibrates in the predetermined frequency range Δf, the vibration anti-node A2 is located at the boundary position B0, or a distance from the boundary position B0 to the vibration anti-node A2 in the longitudinal axis direction is not more than a 1/20 wavelength (λ/20) of the ultrasonic vibration. Therefore, also in the present modification, in the state where the vibrating body unit 20 vibrates in the predetermined frequency region Δf, a distance L2 from the boundary position B0 to the flange portion 36 in the longitudinal axis direction is the same or about the same as a 1/4 wavelength of the ultrasonic vibration.

Furthermore, also in the present modification, a distal end of the vibration generating unit 22 is located on a distal side with respect to the flange portion 36. Consequently, in the state where the vibrating body unit 20 vibrates in the predetermined frequency region Δf, a total length (a distance between the distal end and a proximal end) L5 of the vibration generating unit 22 in the longitudinal axis direction is larger than a 3/4 wavelength of the ultrasonic vibration. Further in the state where the vibrating body unit 20 vibrates in the predetermined frequency region Δf, a distance L6 from the boundary position B0 between the front mass 23 and the elements unit 26 to the distal end of the vibration generating unit 22 (a distal end of the front mass 23) in the longitudinal axis direction is larger than the 1/4 wavelength of the ultrasonic vibration.

In the present modification, between the flange portion 36 and the boundary position B0 in the longitudinal axis direction, the horn 38 is continuous with a proximal side of the flange portion 36, and the block portion 37 is continuous with a proximal side of the horn 38. That is, the horn 38 and the block portion 37 are arranged in a range of the distance L2 between the boundary position B0 and the flange portion 36. Also in the present modification, in the state where the vibrating body unit 20 vibrates in the predetermined frequency region Δf, each of the vibration anti-nodes (A1 to A3) at which stress due to the ultrasonic vibration is zero is not located in the horn 38, and in the horn 38, the stress due to the ultrasonic vibration acts. Consequently, in the horn 38, the amplitude of the ultrasonic vibration is enlarged.

According to the above-mentioned constitution, also in the present modification, an enlargement ratio ε2 of the amplitude at the boundary position B0 is small, and is a minimum value of 1 or a value close to 1, irrespective of a change ratio of an acoustic impedance Z at the boundary position B0. In consequence, a function and an effect similar to those of the first embodiment are produced.

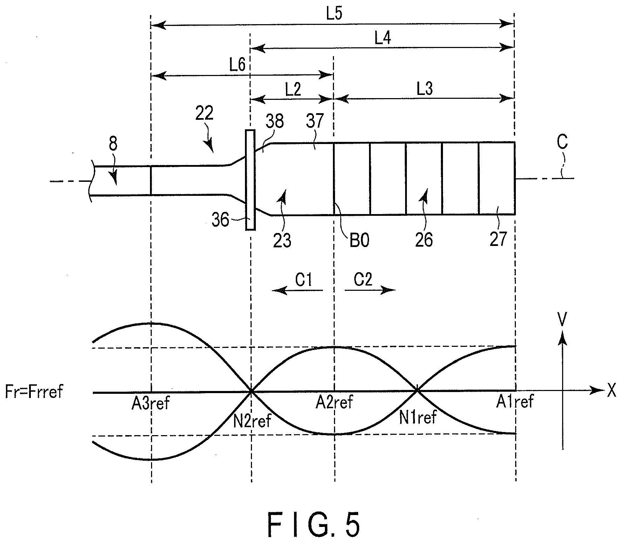

Furthermore, as shown as a second modification in FIG. 5, a flange portion 36 to be supported by a transducer case 21 may be provided at an intermediate position between a distal end (a vibration output end) and a proximal end (a vibration input end) in a horn 38. FIG. 5 shows a graph of a state where a vibrating body unit 20 longitudinally vibrates at a referential resonance frequency Frref in a predetermined frequency range Δf, in addition to a constitution of a vibration generating unit 22. In this graph, the abscissa indicates a position (X) in a longitudinal axis direction and the ordinate shows an amplitude (V) of the longitudinal vibration.

As shown in FIG. 5, also in the present modification, a vibration node N2 of ultrasonic vibration is located in the flange portion 36 in a state where the vibrating body unit 20 vibrates at the referential resonance frequency Frref. Then, in a state where the vibrating body unit 20 vibrates in a predetermined frequency region Δf that is not less than a minimum resonance frequency Frmin and is not more than a maximum resonance frequency Frmax, the vibration node N2 is located in the flange portion 36 or in the vicinity of the flange portion 36. Furthermore, also in the present modification, a vibration anti-node A2 of the ultrasonic vibration is located at a boundary position B0 between a front mass 23 (a block portion 37) and an elements unit 26, in the state where the vibrating body unit 20 vibrates at the referential frequency Frref. Then, in the state where the vibrating body unit 20 vibrates in the predetermined frequency region Δf, the vibration anti-node A2 is located at the boundary position B0 or in the vicinity of the boundary position B0. That is, in the state where the vibrating body unit 20 vibrates in the predetermined frequency range Δf, the vibration anti-node A2 is located at the boundary position B0, or a distance from the boundary position B0 to the vibration anti-node A2 in the longitudinal axis direction is not more than a 1/20 wavelength (λ/20) of the ultrasonic vibration. Therefore, also in the present modification, in the state where the vibrating body unit 20 vibrates in the predetermined frequency region Δf, a distance L2 from the boundary position B0 to the flange portion 36 in the longitudinal axis direction is the same or about the same as a 1/4 wavelength of the ultrasonic vibration.

Furthermore, also in the present modification, a distal end of the vibration generating unit 22 is located on a distal side with respect to the flange portion 36 (the horn 38). Consequently, in the state where the vibrating body unit 20 vibrates in the predetermined frequency region Δf, a total length (a distance between the distal end and a proximal end) L5 of the vibration generating unit 22 in the longitudinal axis direction is larger than a 3/4 wavelength of the ultrasonic vibration. Further in the state where the vibrating body unit 20 vibrates in the predetermined frequency region Δf, a distance L6 from the boundary position B0 between the front mass 23 and the elements unit 26 to the distal end of the vibration generating unit 22 (a distal end of the front mass 23) in the longitudinal axis direction is larger than the 1/4 wavelength of the ultrasonic vibration.

In the present modification, between the flange portion 36 and the boundary position B0 in the longitudinal axis direction, a part of the horn 38 is continuous with a proximal side of the flange portion 36, and the block portion 37 is continuous with a proximal side of the horn 38. Also in the present modification, in the state where the vibrating body unit 20 vibrates in the predetermined frequency region Δf, each of vibration anti-nodes (A1 to A3) at which stress due to the ultrasonic vibration is zero is not located in the horn 38, and in the horn 38, the stress due to the ultrasonic vibration acts. Consequently, in the horn 38, the amplitude of the ultrasonic vibration is enlarged.

According to the above-mentioned constitution, also in the present modification, an enlargement ratio ε2 of the amplitude at the boundary position B0 decreases to a minimum value of 1 or to a value close to 1, irrespective of a change ratio of an acoustic impedance Z at the boundary position B0. In consequence, a function and an effect similar to those of the first embodiment are produced.

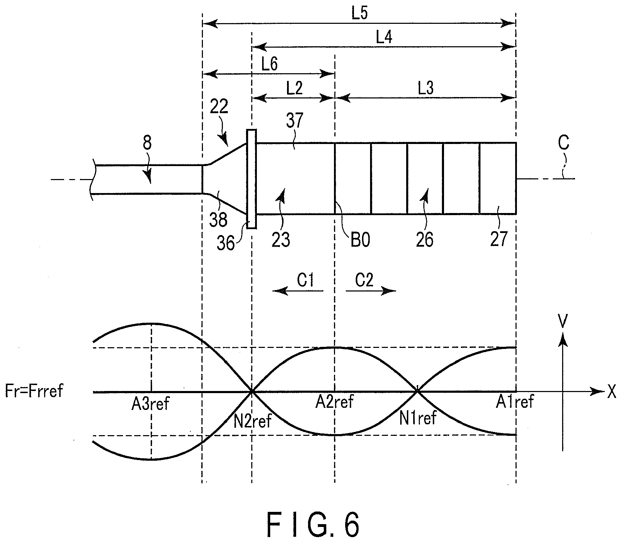

Furthermore, in the above-mentioned embodiment and the like, the vibration anti-node A3 that is one of the vibration anti-nodes of the ultrasonic vibration is located at the distal end of the vibration generating unit 22 in the state where the vibrating body unit 20 vibrates at the referential frequency Frref, but it is not limited to this example. For example, as shown as a third modification in FIG. 6, in a state where a vibrating body unit 20 vibrates at a referential resonance frequency Frref, a vibration anti-node A3 of ultrasonic vibration may be located on a distal side with respect to a distal end of a vibration generating unit 22. FIG. 6 shows a graph of a state where the vibrating body unit 20 longitudinally vibrates at the referential resonance frequency Frref in a predetermined frequency range Δf, in addition to a constitution of the vibration generating unit 22. In this graph, the abscissa indicates a position (X) in a longitudinal axis direction and the ordinate shows an amplitude (V) of the longitudinal vibration.

As shown in FIG. 6, also in the present modification, a vibration node N2 of the ultrasonic vibration is located in a flange portion 36 in a state where the vibrating body unit 20 vibrates at the referential frequency Frref. Then, in a state where the vibrating body unit 20 vibrates in a predetermined frequency region Δf that is not less than a minimum resonance frequency Frmin and is not more than a maximum resonance frequency Frmax, the vibration node N2 is located in the flange portion 36 or in the vicinity of the flange portion 36. Furthermore, also in the present modification, a vibration anti-node A2 of the ultrasonic vibration is located at a boundary position B0 between a front mass 23 (a block portion 37) and an elements unit 26, in the state where the vibrating body unit 20 vibrates at the referential frequency Frref. Then, in the state where the vibrating body unit 20 vibrates in the predetermined frequency region Δf, the vibration anti-node A2 is located at the boundary position B0 or in the vicinity of the boundary position B0. That is, in the state where the vibrating body unit 20 vibrates in the predetermined frequency range Δf, the vibration anti-node A2 is located at the boundary position B0, or a distance from the boundary position B0 to the vibration anti-node A2 in the longitudinal axis direction is not more than a 1/20 wavelength (λ/20) of the ultrasonic vibration. Therefore, also in the present modification, in the state where the vibrating body unit 20 vibrates in the predetermined frequency region Δf, a distance L2 from the boundary position B0 to the flange portion 36 in the longitudinal axis direction is the same or about the same as a 1/4 wavelength of the ultrasonic vibration.

In the present modification, differently from the above-mentioned embodiment and the like, the vibration anti-node A3 is located on the distal side with respect to the distal end of the vibration generating unit 22 also in a state where the vibrating body unit 20 vibrates at any resonance frequency Fr of the predetermined frequency region Δf. Therefore, in the state where the vibrating body unit 20 vibrates in the predetermined frequency region Δf, a distance L6 from the boundary position B0 between the front mass 23 and the elements unit 26 to the distal end of the vibration generating unit 22 (a distal end of the front mass 23) in the longitudinal axis direction is smaller than a half wavelength of the ultrasonic vibration. However, also in the present modification, the distal end of the vibration generating unit 22 is located on the distal side with respect to the flange portion 36 (a horn 38). Consequently, in the state where the vibrating body unit 20 vibrates in the predetermined frequency region Δf, a total length (a distance between the distal end and a proximal end) L5 of the vibration generating unit 22 in the longitudinal axis direction is larger than a 3/4 wavelength of the ultrasonic vibration. Further in the state where the vibrating body unit 20 vibrates in the predetermined frequency region Δf, the distance L6 from the boundary position B0 between the front mass 23 and the elements unit 26 to the distal end of the vibration generating unit 22 (the distal end of the front mass 23) in the longitudinal axis direction is larger than the 1/4 wavelength of the ultrasonic vibration.

Also in the present modification, in the state where the vibrating body unit 20 vibrates in the predetermined frequency region Δf, the vibration anti-node A2 of the ultrasonic vibration is located at the boundary position B0 between the front mass 23 and the elements unit 26 or in the vicinity of the boundary position B0. Consequently, also in the present modification, an enlargement ratio ε2 of the amplitude at the boundary position B0 decreases to a minimum value of 1 or to a value close to 1, irrespective of a change ratio of an acoustic impedance Z at the boundary position B0. In consequence, a function and an effect similar to those of the first embodiment are produced.

Furthermore, in the present modification, also in the state where the vibrating body unit 20 vibrates at any resonance frequency Fr of the predetermined frequency region Δf, the vibration anti-nodes of the ultrasonic vibration which include the vibration anti-node A3 are not located at the distal end of the vibration generating unit 22. Consequently, in a state where a vibration transmitting member 8 is not connected to the vibration generating unit 22 (i.e., in a single body of the vibration generating unit 22), the vibration generating unit 22 does not vibrate in the predetermined frequency region Δf. In consequence, there is effectively prevented a malfunction in which the vibration generating unit 22 vibrates in the state where the vibration transmitting member 8 is not connected.

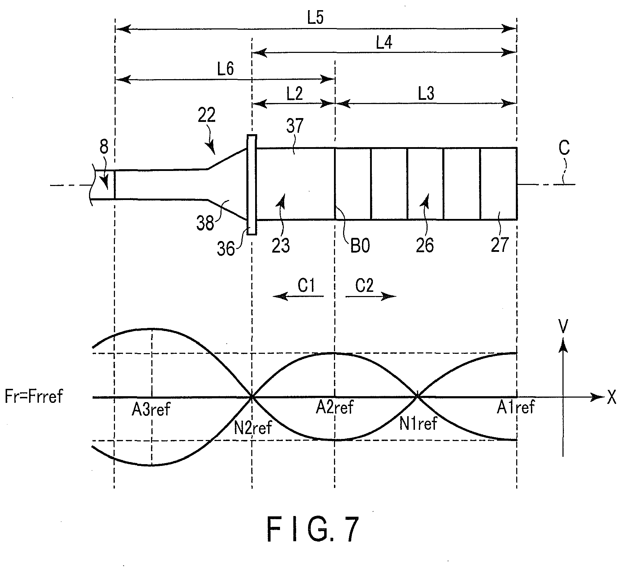

Furthermore, as shown as a fourth modification in FIG. 7, in a state where a vibrating body unit 20 vibrates at a referential resonance frequency Frref, a vibration anti-node A3 of ultrasonic vibration may be located on a proximal side with respect to a distal end of a vibration generating unit 22. FIG. 7 shows a graph of a state where the vibrating body unit 20 longitudinally vibrates at the referential resonance frequency Frref in a predetermined frequency range Δf, in addition to a constitution of the vibration generating unit 22. In this graph, the abscissa indicates a position (X) in a longitudinal axis direction and the ordinate shows an amplitude (V) of the longitudinal vibration.

In the present modification, the vibration anti-node A3 is located on the proximal side with respect to the distal end of the vibration generating unit 22 also in a state where the vibrating body unit 20 vibrates at any resonance frequency Fr of the predetermined frequency region Δf. Therefore, in a state where the vibrating body unit 20 vibrates in the predetermined frequency region Δf, a distance L6 from a boundary position B0 between a front mass 23 and an elements unit 26 to the distal end of the vibration generating unit 22 (a distal end of the front mass 23) in the longitudinal axis direction is larger than a half wavelength of the ultrasonic vibration. Also in the present modification, the distal end of the vibration generating unit 22 is located on a distal side with respect to a flange portion 36 (a horn 38). Consequently, in the state where the vibrating body unit 20 vibrates in the predetermined frequency region Δf, a total length (a distance between the distal end and a proximal end) L5 of the vibration generating unit 22 in the longitudinal axis direction is larger than a 3/4 wavelength of the ultrasonic vibration. Further in the state where the vibrating body unit 20 vibrates in the predetermined frequency region Δf, the distance L6 from the boundary position B0 between the front mass 23 and the elements unit 26 to the distal end of the vibration generating unit 22 (the distal end of the front mass 23) in the longitudinal axis direction is larger than the half wavelength of the ultrasonic vibration as described above, and is larger than a 1/4 wavelength of the ultrasonic vibration.

Also in the present modification, in the state where the vibrating body unit 20 vibrates in the predetermined frequency region Δf, a vibration anti-node A2 of the ultrasonic vibration is located at the boundary position B0 between the front mass 23 and the elements unit 26 or in the vicinity of the boundary position B0. Consequently, also in the present modification, an enlargement ratio ε2 of the amplitude at the boundary position B0 decreases to a minimum value of 1 or to a value close to 1, irrespective of a change ratio of an acoustic impedance Z at the boundary position B0. Therefore, a function and an effect similar to those of the first embodiment are produced.

Furthermore, also in the present modification, similarly to the third modification, vibration anti-nodes of the ultrasonic vibration which include the vibration anti-node A3 are not located at the distal end of the vibration generating unit 22, also in the state where the vibrating body unit 20 vibrates at any resonance frequency Fr of the predetermined frequency region Δf. Consequently, in a state where a vibration transmitting member 8 is not connected to the vibration generating unit 22 (i.e., in a single body of the vibration generating unit 22), the vibration generating unit 22 does not vibrate in the predetermined frequency region Δf. In consequence, there is effectively prevented a malfunction in which the vibration generating unit 22 vibrates in the state where the vibration transmitting member 8 is not connected.

Furthermore, the number of the piezoelectric elements (31A to 31D) is not limited to the above-mentioned embodiment and the like. That is, at least one piezoelectric element (of 31A to 31D) may only be provided in the elements unit 26.

Further in the above-mentioned embodiment and the like, the distance L3 from the proximal end of the vibration generating unit 22 to the boundary position B0 is the same or about the same as the half wavelength of the ultrasonic vibration in the state where the vibrating body unit 20 vibrates in the predetermined frequency region Δf, but it is not limited to this example. For example, in a certain modification, a distance L3 from a proximal end of a vibration generating unit 22 to a boundary position B0 may be the same or about the same as one wavelength of the ultrasonic vibration in a state where a vibrating body unit 20 vibrates in a predetermined frequency region Δf. In this case, a thirdly proximally vibration anti-node A3 among vibration anti-nodes of ultrasonic vibration is located at the boundary position B0 or in the vicinity of the boundary position B0. Then, a thirdly proximally vibration node (N3) among vibration nodes of the ultrasonic vibration is located in a flange portion 36 or in the vicinity of the flange portion 36. Therefore, also in the present modification, similarly to the above-mentioned embodiment and the like, the distance L3 from the boundary position B0 between the block portion 37 and the elements unit 26 to the flange portion 36 in the longitudinal axis direction is the same or about the same as the 1/4 wavelength in the state where the vibrating body unit 20 vibrates in the predetermined frequency region Δf. Then, in the state where the vibrating body unit 20 vibrates in the predetermined frequency range Δf, the vibration anti-node (the referential vibration anti-node) A3 located at the boundary position B0 between the front mass 23 and the elements unit 26 or in the vicinity of the boundary position B0 is closest to the flange portion 36 among the vibration anti-nodes (A1 to A3) located on the proximal side with respect to the flange portion 36.

Furthermore, according to the above-mentioned embodiment and the like, in the state where the vibrating body unit 20 vibrates in the predetermined frequency range Δf, the vibration anti-node (A2; A3) closest to the flange portion 36 among the vibration anti-nodes (A1 and A2; A1 to A3) located on the proximal side with respect to the flange portion 36, but it is not limited to this example. For example, in a certain modification, in a state where a vibrating body unit 20 vibrates in a predetermined frequency range Δf, a thirdly proximally vibration node (N3) among vibration nodes of ultrasonic vibration is located in a flange portion 36 or in the vicinity of the flange portion 36, and three vibration anti-nodes (A1 to A3) are located on the proximal side with respect to the flange portion 36. Then, according to the present modification, in the state where the vibrating body unit 20 vibrates in the predetermined frequency range Δf, a referential vibration anti-node, which is the vibration anti-node (A2) secondly close to the flange portion 36 among the vibration anti-nodes (A1 to A3) located on the proximal side with respect to the flange portion 36, is located at a boundary position B0 between a front mass 23 and an elements unit 26 or in the vicinity of the boundary position B0. Therefore, the vibration anti-node (A3) closest to the flange portion 36 among the vibration anti-nodes (A1 to A3) located on the proximal side with respect to the flange portion 36 is located away from the boundary position B0 between the front mass 23 and the elements unit 26. Also in the present modification, the vibration anti-node (the referential vibration anti-node) A2 located at the boundary position B0 between the front mass 23 and the elements unit 26 or in the vicinity of the boundary position B0 is one of the vibration anti-nodes (A1 to A3) located on the proximal side with respect to the flange portion 36.

Furthermore, in a certain modification, a flange portion 36 and a block portion 37 are only provided in a front mass 23, and a horn 38 is not provided. In this case, in the front mass 23, an amplitude of ultrasonic vibration is not enlarged.

Furthermore, in the ultrasonic treatment instrument 2, the ultrasonic vibration is transmitted to the treatment section 17 of the vibration transmitting member 8, and high frequency electric power is supplied from the energy source unit 10 to the treatment section 17 and the jaw 7, and the treatment section 17 and the jaw 7 may function as electrodes of the high frequency electric power. When the treatment section 17 and the jaw 7 function as the electrodes, a high frequency current flows through a treated target gripped between the jaw 7 and the treatment section 17, the treated target is denatured, and coagulation is promoted. In this case, the high frequency electric power is supplied to the treatment section 17 through the bolt portion 25, the front mass 23 and the vibration transmitting member 8, but the front mass 23 and the bolt portion 25 are electrically isolated from the piezoelectric elements (31A to 31D), and the high frequency electric power to be supplied to the treatment section 17 is not supplied to the piezoelectric elements (31A to 31D). However, also in this case, the current (the alternating current) to generate the ultrasonic vibration is supplied to the piezoelectric elements (31A to 31D).

Furthermore, in the ultrasonic treatment instrument 2, the jaw 7 does not have to be provided. In this case, for example, the treatment section 17 projecting from the distal end of the sheath 6 is formed into a hook shape. In a state where the treated target is hooked to a hook, the treatment section 17 is vibrated by the ultrasonic vibration, thereby incising the treated target.

In the above-mentioned embodiment and the like, a vibration generating unit (22) includes a front mass (23) which is capable of transmitting ultrasonic vibration, and in the front mass (23), there are provided a flange portion (36) supported by a housing case (21), and a block portion (37) provided on a proximal side with respect to the flange portion (36). A distal end of an elements unit (26) abuts on a proximal end of the block portion (37), and the elements unit (26) includes piezoelectric elements (31A to 31D) to which electric power is supplied so as to generate the ultrasonic vibration. The ultrasonic vibration generated in the piezoelectric elements (31A to 31D) is transmitted from the proximal side to a distal side through the front mass (23), whereby a vibrating body unit (20) including the front mass (23) and the elements unit (26) vibrates in a predetermined frequency region (Δf) where a referential vibration anti-node (A2; A3), which is one of vibration anti-nodes (A1 and A2; A1 to A3) located on the proximal side with respect to the flange portion (36), is located at a boundary position (B0) between the block portion (37) and the elements unit (26) or in the vicinity of the boundary position (B0).

Although the embodiments, etc. of the present invention have been described above, the present invention is not limited to the above-described embodiments, etc., and, needless to say, various modifications can be made without departing from the spirit of the invention.

## Claims

1. A vibration generating unit comprising:
a front mass which includes a flange portion supported by a housing case, and a block portion provided on a proximal side with respect to the flange portion, the front mass being capable of transmitting ultrasonic vibration; and
an element unit which includes a piezoelectric element to which electric power is supplied so as to generate the ultrasonic vibration, and whose distal end abuts on a proximal end of the block portion, the element unit being configured to transmit the ultrasonic vibration generated in the piezoelectric element from the proximal side to a distal side through the front mass, thereby vibrating the front mass in a predetermined frequency region where a referential vibration anti-node, which is one of vibration anti-nodes located on the proximal side with respect to the flange portion, is located at a boundary position between the block portion and the element unit or in the vicinity of the boundary position.

2. The vibration generating unit of claim 1,
wherein in a state where the front mass and the element unit are vibrated in the predetermined frequency region by the ultrasonic vibration, a distance from the boundary position to the referential vibration anti-node in a longitudinal axis direction is zero or is not more than a 1/20 wavelength of the ultrasonic vibration.

3. The vibration generating unit of claim 1,
wherein the front mass includes a horn which is provided on the distal side with respect to the boundary position between the block portion and the element unit, and which is configured to enlarge an amplitude of the ultrasonic vibration transmitted toward the distal side.

4. The vibration generating unit of claim 1,
wherein a distal end of the front mass forms a distal end of the vibration generating unit, and
in a state where the front mass and the element unit are vibrated in the predetermined frequency region by the ultrasonic vibration, a distance from the referential vibration anti-node to the distal end of the front mass in a longitudinal axis direction is larger than a 1/4 wavelength of the ultrasonic vibration.

5. The vibration generating unit of claim 1,
wherein in a state where the front mass and the element unit are vibrated in the predetermined frequency region by the ultrasonic vibration, a total length of the vibration generating unit in a longitudinal axis direction is larger than a 3/4 wavelength of the ultrasonic vibration.

6. A vibrating body unit comprising:
the vibration generating unit of claim 1; and
a vibration transmitting member which includes a treatment section in a distal portion thereof, and whose proximal end is connected to a distal end of the front mass, the ultrasonic vibration generated in the element unit being transmitted to the vibration transmitting member through the front mass, and the vibration transmitting member being configured to transmit the ultrasonic vibration transmitted from the vibration generating unit toward the treatment section.

7. An ultrasonic treatment instrument comprising:
the vibrating body unit of claim 6;
the housing case to which the vibration generating unit is attached in a state of supporting the flange portion; and
a held unit from which the vibration transmitting member extends toward the distal side, and which is holdable.
